# EUROPÄISCHE PATENTSCHRIFT

(11) **EP 1 233 984 B1**
(45) Veröffentlichungstag und Bekanntmachung des Hinweises auf die Patenterteilung: **24.11.2004**
(21) Anmeldenummer: 00987296.1
(22) Anmeldetag: 27.11.2000
(51) Int. Cl.: C07K 14/435, A61K 38/17, G01N 33/68, A61P 37/08

(54) **VERFAHREN ZUR HERSTELLUNG VON WESPENGIFT-ALLERGENEN MIT VERMINDERTER IgE-REAKTIVITÄT**
PROCESS FOR PRODUCING WASP POISON ALLERGENS WITH REDUCED IgE-REACTIVITY
PROCEDE POUR LA PREPARATION DES ALLERGENS DES TOXINS DE LA GUEPE AYANT UNE REACTIVITE IgE REDUITE

(30) Priorität: 01.12.1999 DE 19957904
(43) Veröffentlichungstag der Anmeldung: 28.08.2002
(73) Patentinhaber: MERCK PATENT GmbH, 64293 Darmstadt (DE); Suck, Roland, 22303 Hamburg (DE)
(72) Erfinder: SUCK, Roland, 22303 Hamburg (DE); CROMWELL, Oliver, 21465 Wentorf (DE); FIEBIG, Helmut, 21493 Schwarzenbek (DE)
(86) Internationale Anmeldenummer: PCT/EP2000/011776
(87) Internationale Veröffentlichungsnummer: WO 2001/040266

(56) Entgegenhaltungen:
- KING T.P. ET AL.: "Murine T and B cell response to natural and recombinant hornet venom allergen Dol m 5.02 and its recombinant fragments" THE JOURNAL OF IMMUNOLOGY, Bd. 154, 1995, Seiten 577-584, XP002173770
- SUCK R. ET AL.: "Purification and immunobiological characterization of folding variants of the recombinant major wasp allergen ves v 5 (antigen 5)" INTERNATIONAL ARCHIVES OF ALLERGY AND IMMUNOLOGY, Bd. 121, 1. April 2000 (2000-04-01), Seiten 284-291, XP001010205
- MONSALVE R.I. ET AL.: "Expressions of recombinant venom allergen, antigen 5 of yellowjacket (Vespula vulgaris) and paper wasp (Polistes annularis), in bacteria and yeast" PROTEIN EXPRESSION AND PURIFICATION, Bd. 16, August 1999 (1999-08), Seiten 410-416, XP002173771 in der Anmeldung erwähnt
- SOLDATOVA L.N. ET AL.: "Superior biologic activity of the recombinant bee venom allergen hyaluronidase expressed in baculovirus-infected insect cells as compared with Escherichia coli" THE JOURNAL OF ALLERGY AND CLINICAL IMMUNOLOGY, Bd. 101, Nr. 5, Mai 1998 (1998-05), Seiten 691-698, XP001014760 in der Anmeldung erwähnt
- FÖRSTER E. ET AL.: "Natural and recombinant enzymatically active or inactive bee venom phospholipase A2 has the same potency to release histamine from basophils in patients with hymenoptera allergy" THE JOURNAL OF ALLERGY AND CLINICAL IMMUNOLOGY, Bd. 95, Nr. 6, Juni 1995 (1995-06), Seiten 1229-1235, XP001014781

## Beschreibung

Die Erfindung betrifft ein Verfahren zur Herstellung von rekombinanten Wespengift-Allergen Antigen 5 wobei sich besagte Allergene je nach Durchführung des Herstellungsverfahrens durch naturidentische oder naturfremde Faltungen (Konformationen) unterscheiden lassen.

Eine Anwendung für Faltungsformen, die dem natürlichen Molekül entsprechen, besteht in der Einzelallergen-differenzierten Diagnostik *(in vitro* oder *in vivo)* von Allergikern, speziell Insektengiftallergikern.

Die naturfremden Faltungsformen können als nebenwirkungsarme Therapeutika zur spezifischen Immuntherapie eingesetzt werden. Damit könnten diese rekombinanten Faltungsvarianten eine sicherere Behandlung als der Naturstoff bewirken. Das Verfahren ist so konzipiert, daß eine biotechnologische Herstellung unter Bedingungen, die für Pharmazeutika notwendig sind (GMP), durchgeführt werden kann.

Insektenstichallergien werden hauptsächlich von Wespen und Honigbienen verursacht und können zu schweren systemischen Symptomen bis hin zur potenziell tödlich verlaufenden Anaphylaxie führen (Müller, U.R., in: Insect sting allergy, Gustav Fischer Verlag; 1990). Bei den Typ 1-Allergie auslösenden Substanzen handelt es sich um Proteine, Glykoproteine oder Polypeptide des Insektenvenoms. Diese Allergene reagieren nach Injektion mit den bei sensibilisierten Personen an der Oberfläche von Mastzellen gebundenen IgE-Molekülen. Werden solche FcεRI-gebundenen IgE-Moleküle durch ein Allergen miteinander vernetzt, führt dies zur Ausschüttung von Mediatoren (z.B. Histamin, Leukotriene) und Zytokinen durch die Effektorzelle und damit zu den entsprechenden klinischen Symptomen.

Als allergene Bestandteile des Bienenvenoms wirken neben dem Peptid Melittin die Enzyme Hyaluronidase und Phospholipase A2 (Habermann, E, 1972, Science 177, 314-322). Im Fall der Wespe kommen als enzymatisch aktive Hauptallergene ebenfalls eine Hyaluronidase, die der des Bienenvenoms ähnlich ist (Hoffmann, D.R., 1986, J. Allergy Clin. Immunol. 78, 337-343) und eine Phospholipase A1 vor. Das wichtigste Hauptallergen des Wespengiftes ist das Antigen 5, für das bisher keine enzymatische Aktivität nachgewiesen werden konnte (King et al., 1978, Biochemistry 17, 5165-5174). Sämtliche der genannten Allergene sind bereits molekularbiologisch charakterisiert und die entsprechenden cDNA-Moleküle kloniert (u.a. Fang et al., 1988, PNAS, 895-899; Soldatova et al., 1993, FEBS, 145-149; Kuchler et al., 1989, Eur. J. Biochem. 184, 249-254). Mit Hilfe von cDNA Sequenzen ist es möglich, rekombinante Allergene herzustellen, die in der Diagnostik und Therapie von Allergien Verwendung finden könnten (Scheiner and Kraft, 1995, Allergy 50, 384-391).

Im Zusammenhang mit der vorliegenden Erfindung ist das Hauptallergen Antigen 5 von besonderer Bedeutung, da sich die Erfindung dieses Moleküls bedient. Es handelt sich dabei um ein ca. 25 kDa großes, nicht glykosyliertes Protein. Die Primärsequenz beinhaltet 8 Cysteinreste, was auf vier Disulfidbrükken hinweist (Hoffman, D.R., 1993, J. Allergy Clin Immunol. 92: 707-716). Ein klassischer Ansatz zur wirksamen therapeutischen Behandlung von Insektengiftallergien stellt die Spezifische Immuntherapie oder Hyposensibilisierung dar (Müller, U.R., in: Insect sting allergy, Gustav Fischer Verlag; 1990). Dabei werden dem Patienten natürliche Allergenextrakte in steigenden Dosen subkutan injiziert. Allerdings besteht bei dieser Methode die Gefahr von allergischen Reaktionen bis zum anaphylaktischen Schock. Da gerade im Fall von Insektenstichhyposensibilisierungen mit starken Reaktionen zu rechnen ist, wird zur Zeit ausschließlich stationär behandelt.

Eine Therapieoptimierung wäre mit rekombinant hergestellten Allergenen besonders bei der Insektenstichallergie möglich. Definierte, ggf. auf individuelle Sensibilisierungsmuster der Patienten abgestimmte Cocktails von hochreinen rekombinant hergestellten Allergenen (Scheiner and Kraft, 1995) könnten Extrakte aus natürlichen Allergenquellen ablösen. Realistische Perspektiven, die zu einer sicheren Hyposenibilisierung mit solchen rekombinanten Allergenen führen können, bieten gezielt mutierte rekombinante Allergene, bei denen IgE-Epitope spezifisch deletiert werden, ohne die für die Therapie essenziellen T-Zell Epitope zu beeinträchtigen (Schramm et al., 1999, J. Immunol. 162, 2406-2414). Von der heterologen Expression in *E*.*coli* ist bekannt, daß die meisten eukaryontischen Proteine nicht oder nur in geringem Ausmaß die 'natürliche' Konformation annehmen. Eine Folge von diesen Fehlfaltungen ist häufig die Unlöslichkeit dieser Proteine. Dies ist besonders bei cysteinhaltigen Proteinen zu beobachten (Kuchier et al., 1989, Eur. J. Biochem. 184, 249-254). Insbesondere von Antigen 5 wurde berichtet, daß die Expression in Bakterien zu unlöslichen Aggregaten führt, die nicht die natürliche Konformation besitzen (Monsalve et al., 1999, Protein Express Purif. 16(3): 410-416). Solche unlöslichen Aggregate sind weder zur Diagnostik noch zur Therapie nutzbar. King et al. []. Immunol. 154(1995) 577-584) Beschreiben eine Methde zur rekombonanten Herstellung von Hornissengift Allergen Vol nr 5.02 in Bakterien.

Häufig werden die in *E. coli* unlöslichen Proteine für Forschungszwecke in einem eukaryontischen Expressionssystem dargestellt, wie z.B. Hefe oder Insektenzellen (Monsalve et al., 1999, Protein Express Purif 16(3): 410-416; Soldatova et al., 1998, J Allergy Clin Immunol 101:691-698). Nachteile der eukaryontischen Expressionssysteme stellen jedoch vor allem mögliche Hyperglykosylierungen (Grobe et al., 1999, Eur J Biochem 263:33-40), proteolytische Degradationsprozesse und vergleichsweise kleine Produktausbeuten dar (Glover and Hames (eds.), 1995, Expression Systems, IRL Press, Oxford-New York-Tokyo). Für die allergologische Anwendung im Sinne einer pharmazeutisch-medizinischen Diagnostik und Therapie sind solche Proteine deshalb meist ungeeignet.

Die Produkte des erfindungsgemäßen Verfahrens, welche die natürliche Konformation besitzen, können in der In-vitro- und In-vivo-Diagnostik von Wespenstichallergien, vorteilhaft angewendet werden. Diese naturidentische Faltungsform steht zur Detektion von IgE-Antikörpern in etablierten Verfahren zur Verfügung.

Andererseits können die mit Hilfe der Erfindung hergestellten Varianten, die sich durch eine im wesentlichen nicht oder nur partiell IgE-reaktive Konformationen auszeichnen, als hypoallergene Komponenten in Präparaten zur spezifischen Immuntherapie angewendet werden. Unter dem Ausdruck "hypoallergen" wird oben und unten erfindungsgemäß eine verminderte bis fehlende, vorzugsweise um 5 bis 95 %, insbesondere 20 bis 85 %, verminderte Allergenität (gegenüber dem natürlichen Allergen) verstanden, welche durch eine verminderte IgE-Antwort bedingt ist.

Bei der vorliegenden Erfindung handelt es sich um eine Methode, mit der rekombinante Allergene in Bakterien (*E*.*coli*) hergestellt werden können. Durch hohe Anreicherung der unlöslichen Protein-Aggregate findet ein erster Reinigungsschritt statt. Diese Aggregate werden dann ohne Zusatz von Reduktionsmitteln denaturiert. In Abhängigkeit von den folgenden Dialysebedingungen werden unterschiedliche Faltungsformen erhalten. Entscheidend ist, daß es sich dabei um monomere und lösliche Moleküle handelt. Die eine lösliche Faltungsvariante besitzt eine dem natürlichen Allergen vergleichbare IgE-Reaktivität und kann demnach für diagnostische Zwecke verwendet werden. Eine solches Produkt wird durch Dialyse mit Cystein-haltiger Lösung erhalten.

Die anderen alternativen löslichen Faltungsvarianten sind strukturell verschieden von dem natürlichen Allergen und zeichnen sich durch verminderte oder fehlende IgE-Reaktivität aus. Aus diesem Grund sind solche Varianten geeignet, um eine verbesserte Immuntherapie zu ermöglichen. Ein solches hypoallergenes Produkt wird erfindungsgemäß durch Dialyse mit sauren Puffern, vorzugsweise in einem pH-Wert-Bereich zwischen 3,5 und 6,5, insbesondere zwischen 4,0 und 5,5 gewonnen.

Das nach einem erfindungsgemäßen Verfahren hergestellte rekombinante Wespengiftallergen, Antigen 5 ist dadurch gekennzeichnet, daß es eine verminderte IgE-Reaktivität, bzw. Allergenität besitzt. Erfindungsgemäß ist die Allergenität dieser Proteine bis zu 95%, im Vergleich zu dem natürlichen Allergen reduziert.

Erfindungsgemäß bevorzugt ist ein entsprechendes rekombinantes Wespen-Insektenallergen Antigen 5, aus *Vespula vulgaris* und *Vespula germanica*.

Ferner ist Gegenstand der Erfindung ein Verfahren zur Gewinnung von im wesentlich reinen rekombinanten Wespengiftallergenen Antigen 5 mit verminderter Allergenität bzw. IgE-Reaktivität, dadurch gekennzeichnet, daß
- die allergenen Proteine in Bakterienzellen in unlöslicher Form als "inclusion bodies" hergestellt werden,
- besagte unlösliche Aggregate ohne Zusatz von Reduktionsmitteln denaturiert werden,
- die denaturierten Produkte durch Dialyse unter Verwendung saurer Puffer in lösliche, monomere Allergene überführt werden und
- eine abschließende Dialyse gegen destilliertes Wasser durchgeführt wird.
Vorzugsweise erfolgt besagte Denaturierung mit Guanidinium-Chlorid. Vorzugsweise werden zur Dialyse saure Puffer, vorzugsweise Natriumacetat-Puffer mit einem pH-Wert zwischen 4.5 und 5,0, eingesetzt.

Gegenstand der Erfindung ist aber auch ein Verfahren zur Gewinnung von rekombinanten wespen giftallergenen mit normaler Allergenität, bzw. IgE-Reaktivität, wobei bei der ersten Dialyse Cystein-haltige Lösungen anstelle saurer Puffer eingesetzt werden.

Gegenstand der Erfindung ist ferner eine pharmazeutische Zubereitung, welche ein entsprechendes rekombinantes Allergen mit verminderter, bzw. abgeschwächter IgE-Reaktivität sowie entsprechende Hilfs- und Trägerstoffe enthält.

Letztlich ist Gegenstand der Erfindung die Verwendung von Wespengiftallergene Antigen 5 mit normaler Allergenität bzw. IgE-Reaktiontät, erhältlich nach einem entsprechenden oben oder unten beschriebenen Verfahren zur in-vitro Diagnose von Wespenstichallergien.

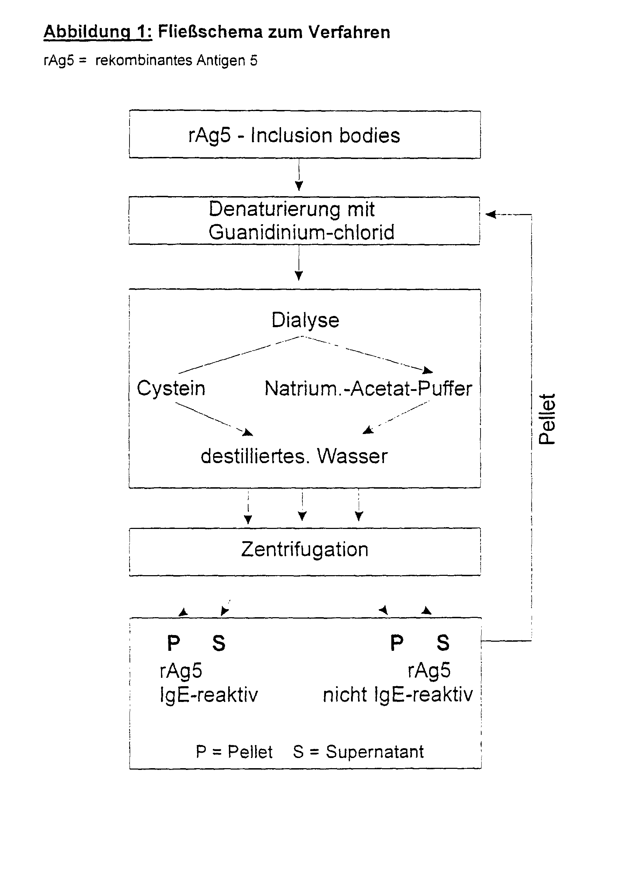

Im nachfolgenden wird das Verfahren im Detail beschrieben:
Beispielhaft wurden die Wespengiftallergene Antigen 5 von *Vespula vulgaris* (Ves v 5) und Antigen 5 von *Vespula germanica* (Ves g 5) in den Expressionsvektor pSE420 kloniert und in den K12-Bakterienstamm M15 pREP4 transformiert. Ein Fließschema des Verfahrens findet sich in Abbildung 1.

Zur Herstellung der rekominanten Allergene wird eine Vorkultur des Stammes zum Animpfen einer Expressionskultur genutzt. Die Expression, durch IPTG induziert, findet in einem Schikanekolben bei 37°C in LB-Medium und limitierter Sauerstoffversorgung statt (90 rpm/min). Die Bakterien werden nach 5 stündiger Expression durch Zentrifugation geerntet (5000xg, 10 min, 20°C). Der Bakterienaufschluß erfolgt nach Resuspension der Zellen in Puffer (50mM Tris/HCl, 25% (w/v) Sucrose, pH 8.0) durch Lysozymzugabe (10µg/g Naßgewicht). Es folgt die Zugabe des gleichen Volumens von Detergenzlösung (0.2 M NaCl, 1%(w/v) DOC, 1%(w/v) Nonidet P40). Diese Aufschlußlösung wird anschließend mit Ultraschall behandelt (3 min auf Eis, 130 Watt, 0.5 s Impuls). Da die Expressionsprodukte primär als unlösliche Aggregate (Einschlusskörper, 'inclusion bodies') vorliegen, können sie aufgrund ihrer hohen Dichte von einem Großteil der übrigen Komponenten (Zellwandfragmente, Ribosomen etc.) durch Zentrifugation bei 3000 x g abgetrennt werden. Die weitere Reinigung erfolgt durch drei aufeinanderfolgende Waschschritte mit detergenzhaltigen Lösungen (1 % Triton X-100). Im Anschluß werden die gereinigten Einschlusskörper durch Zugabe von Denaturierungspuffer (6M Guanidinium-Chlorid, 20 mM Tris/HCl, pH 8.0) aufgeschlossen und 2 h bei RT geschüttelt.

Zur Gewinnung von IgE-reaktiven Faltungsformen wird der Denaturierungsansatz in einen Dialyseschlauch (Ausschlussgrenze 12-14 kDa) gefüllt und gegen das 100 fache Volumen Cysteinlösung (5mM Cystein) 12 h unter Rühren bei RT dialysiert. Im Anschluß erfolgt eine Dialyse gegen destilliertes Wasser, um das Cystein zu entfernen.

Um Konformationen mit verminderter IgE-Reaktivität zu erhalten, wird die erste Dialyse gegen 20 mM Natriumacetat-Puffer (pH 5.0) durchgeführt werden. Auch hier erfolgt eine weiterer Dialyse gegen destilliertes Wasser. Nach Entnahme werden die wasserlöslichen Allergene durch Zentrifugation von den ausgefallenen Aggregaten abgetrennt. Der Überstand enthält die gewünschten löslichen rekombinanten Allergene. Anstelle von Natriumacetat-Puffer können auch andere saure Puffer verwendet werden, welche in einem Bereich von 3,5 bis 6,5, vorzugsweise 4,0 und 5,5 zu puffern vermögen. Beispiele solcher Puffersysteme sind in der Literatur hinlänglich beschrieben.

Die bei beiden Methoden anfallenden präzipitierten rekombinanten Allergene können nochmals denaturiert und nach dem gleichen Schema behandelt werden. Dadurch wird die Ausbeute deutlich erhöht.

Nach den Dialyseschritten sind die Produkte zu ca. 95% rein. Weitere Reinigungsschritte der basischen Insektengiftallergene sind Kationenaustauschchromatographie (Puffer pH 7.2) mit z.B. Source S (Pharmacia, Freiburg, Germany) und Gelfiltration. Die Gelfiltration dient neben der Abtrennung von hochund niedermolekularen Minimalverunreinigungen auch zur Entsalzung.

Die Qualitätskontrollen der Produkte basieren auf folgenden charakteristischen Eigenschaften, die tabellarisch für Antigen 5 zusammengestellt sind: nAntigen = natürliches Antigen

| **Eigenschaft** | **Faltung mit natürlicher IgE- Reaktivität** | **Faltung mit verminderter IgE-Reaktivität** |
|---|---|---|
| App. MW in der SDS-PAGE (nicht reduzierende Bed.) | 25 kDa | 26-27 kDa |
| Salzkonz. zur Elution in der Source S | 320 mM NaCl | 400 mM NaCl |
| Spaltung mit Protease V8 | 15 kDa Fragment + Peptide | Peptide < 10 kDa |
| Antigen 5 spezifische monoklonale Antikörper | Detektion durch 8E3, 1E11 | Detektion nur mit 8E3 möglich |
| Frequenz IgE-Reaktivität mit Allergikerseren | > 95% | <10% |
| Allergene Potenz | ähnlich nAntigen 5 | >10 x geringer als nAg5 |

Die verwendeten Aufreinigungstechniken sowie rekombinante Klonierungs und Expressionstechniken sind dem Fachmann bekannt und zugänglich und können durch bekannte ähnliche Verfahrenstechniken ersetzt werden.

## Patentansprüche

1. Verfahren zur Gewinnung von im wesentlich reinen rekombinanten Wespengiftallergenen Antigen 5 mit verminderter Allergenität bzw. IgE-Reaktivität, **dadurch gekennzeichnet, daß**
- die allergenen Proteine in Bakterienzellen in unlöslicher Form als "inclusion bodies" hergestellt werden,
- besagte unlösliche Aggregate ohne Zusatz von Reduktionsmitteln denaturiert werden,
- die denaturierten Produkte durch Dialyse unter Verwendung saurer Puffer in lösliche, monomere Allergene überführt werden und
- eine abschließende Dialyse gegen destilliertes Wasser durchgeführt wird.

2. Verfahren nach Anspruch 1, **dadurch gekennzeichnet, daß** die Denaturierung mit Guanidinium-Chlorid erfolgt.

3. Verfahren nach Anspruch 1 oder 2, **dadurch gekennzeichnet, daß** bei der Dialyse Puffer mit einem pH-Wert zwischen 3,5 und 6,5 eingesetzt werden.

4. Verfahren nach einem oder mehreren der Ansprüche 1 bis 3, **dadurch gekennzeichnet, daß** Allergene der Spezies Vespula spec., insbesondere Vespula vulgaris und Vespula germanica und Paravespula spec. verwendet werden.

5. Pharmazeutische Zubereitung, enthaltend ein rekombinantes Allergen, hergestellt nach einem der in den Ansprüchen 1 bis 4 beschriebenen Verfahren, sowie entsprechende Hilfs- und Trägerstoffe.

6. Verwendung von Wespengiftallergen Antigen 5, erhältlich nach einem der in den Ansprüchen 1 bis 4 beschriebenen Verfahren, zur Herstellung eines Arzneimittels zur spezifischen Immuntherapie bzw. Hyposensibilisierung von Wespengiftallergikern.

7. Verfahren zur Gewinnung von rekombinanten Wespengiftallergenen Antigen 5 mit normaler Allergenität bzw. IgE-Reaktivität **dadurch gekennzeichnet, daß** die Verfahrensschritte gemäß einem oder mehreren der Ansprüche 1 oder 2 durchgeführt werden, bei der ersten Dialyse jedoch anstelle saurer Puffer cystein-haltige Lösungen eingesetzt werden.

8. Verfahren nach Anspruch 7, **dadurch gekennzeichnet, daß** Allergene der Spezies Vespula spec., insbesondere Vespula vulgaris und Vespula germanica und Paravespula spec. verwendet werden.

9. Verwendung von Wespengiftallergen Antigen 5, erhältlich nach einem der in den Ansprüchen 7 oder 8 beschriebenen Verfahren, zur in-vitro Diagnose von Wespenstichallergien.

## Claims

1. Process for obtaining essentially pure recombinant wasp venom allergen antigen 5 having reduced allergeneity or IgE reactivity, **characterised in that**
- the allergenic proteins are prepared in insoluble form as inclusion bodies in bacterial cells,
- the said insoluble aggregates are denatured without addition of reducing agents,
- the denatured products are converted into soluble, monomeric allergens by dialysis using acidic buffers, and
- final dialysis against distilled water is carried out.

2. Process according to Claim 1, **characterised in that** the denaturing is carried out with guanidinium chloride.

3. Process according to Claim 1 or 2, **characterised in that** buffers having a pH of between 3.5 and 6.5 are employed in the dialysis.

4. Process according to one or more of Claims 1 to 3, **characterised in that** allergens of the species Vespula spp., in particular Vespula vulgaris and Vespula germanica, and Paravespula spp. are used.

5. Pharmaceutical preparation comprising a recombinant allergen prepared by one of the processes described in Claims 1 to 4, and corresponding adjuvants and excipients.

6. Use of wasp venom allergen antigen 5 obtainable by one of the processes described in Claims 1 to 4 for the preparation of a medicament for the specific immunotherapy or hyposensitisation of wasp venom allergy sufferers.

7. Process for obtaining recombinant wasp venom allergen antigen 5 having normal allergeneity or IgE reactivity, **characterised in that** the process steps according to one or more of Claims 1 or 2 are carried out, but cysteine-containing solutions are employed instead of acidic buffers in the first dialysis.

8. Process according to Claim 7, **characterised in that** allergens of the species Vespula spp., in particular Vespula vulgaris and Vespula germanica, and Paravespula spp. are used.

9. Use of wasp venom allergen antigen 5 obtainable by one of the processes described in Claim 7 or 8 for the in-vitro diagnosis of wasp sting allergies.

## Revendications

1. Procédé pour obtenir l'allergène antigène 5 de venin de guêpe recombinant essentiellement pur présentant une alergénéité réduite ou une réactivité IgE réduite, **caractérisé en ce que**
- les protéines allergéniques sont préparées sous une forme soluble en tant que corps d'inclusion dans des cellules de bactéries,
- lesdits agrégats insolubles sont dénaturés sans addition d'agent réducteur,
- les produits dénaturés sont convertis selon des allergènes monomériques solubles par dialyse en utilisant des tampons acides, et
- une dialyse finale contre de l'eau distillée est effectuée.

2. Procédé selon la revendication 1, **caractérisé en ce que** la dénaturation est effectuée avec du chlorure de guanidium.

3. Procédé selon la revendication 1 ou 2, **caractérisé en ce que** des tampons ayant un pH entre 3,5 et 6,5 sont employés dans la dialyse.

4. Procédé selon une ou plusieurs des revendications 1 à 3, **caractérisé en ce que** des allergènes des espèces Vespula spp., en particulier Vespula vulgaris et Vespula germanica, et des espèces Paravespula spp. sont utilisés.

5. Préparation pharmaceutique comprenant un allergène recombinant préparé par l'un des procédés décrits dans les revendications 1 à 4, et adjuvants et excipients correspondants.

6. Utilisation d'allergène antigène 5 de venin de guêpe pouvant être obtenu par l'un des procédés décrits dans les revendications 1 à 4 pour la préparation d'un médicament pour l'immunothérapie ou hyposensibilisation spécifique de personnes souffrant d'allergie au venin de guêpe.

7. Procédé pour obtenir l'allergène antigène 5 de venin de guêpe recombinants présentant une alergénéité réduite ou une réactivité IgE réduite, **caractérisé en ce que** les étapes de procédé selon une ou plusieurs des revendications 1 ou 2 sont mises en oeuvre, sauf que des solutions contenant de la cystéine sont employées à la place des tampons acides dans la première dialyse.

8. Procédé selon la revendication 7, **caractérisé en ce que** des allergènes des espèces Vespula spp., en particulier Vespula vulgaris et Vespula germanica, et des espèces Paravespula spp. sont utilisés.

9. Utilisation d'allergène antigène 5 de venin de guêpe pouvant être obtenu par l'un des procédés décrits dans la revendication 7 ou 8 pour le diagnostic in vitro d'allergies à la piqûre de guêpe.
